# EUROPEAN PATENT APPLICATION

(11) **EP 4 781 978 A2**
(43) Date of publication of application: **29.07.2026**
(21) Application number: 26170190.8
(22) Date of filing: 01.05.2019
(51) Int. Cl.: A61K 9/00

(54) **EYE TREATMENTS EMPLOYING SERUM FROM WHOLE BLOOD**

(30) Priority: 01.05.2018 US 201862664939 P; 20.09.2018 US 201862733918 P
(62) Divisional of application: 19796766.4
(71) Applicant: Muller, David, Boston, MA 02111 (US)
(72) Inventor: Muller, David, Boston, MA 02111 (US)
(74) Representative: Mewburn Ellis LLP

(57) **Abstract**

Eye drops for treating dry eye include serum extracted from whole blood. An example method includes collecting whole blood from one or more donors (e.g., allogenic whole blood); separating the whole blood to obtain a serum; applying radiation to the serum for viral inactivation; and adding one or more additives to the serum to produce eye drops.

## Description

### CROSS REFERENCE TO RELATED APPLICATIONS

This application claims priority to and the benefit of U.S. Provisional Patent Application No. 62/664,939, filed May 1, 2018, and U.S. Provisional Patent Application No. 62/733,918, filed September 20, 2018, the contents of these applications being incorporated entirely herein by reference.

### BACKGROUND

### Field

The present disclosure pertains to eye treatments, and more particularly, to treatments of dry eye, *e.g.,* eye drops, that employ serum extracted from allogenic whole blood.

### Description of Related Art

Dry eye occurs when the quantity and/or quality of tears fails to keep the surface of the eye adequately lubricated. In a healthy eye, lubricating tears called basal tears continuously bathe the cornea. With every blink of the eye, basal tears flow across the cornea, nourishing its cells and providing a layer of liquid protection against the environment. When the glands nearby each eye fail to produce enough basal tears, or when the composition of the tears changes, the health of the eye and vision are compromised. Vision may be affected, for instance, because tears on the surface of the eye play an important role in focusing light.

Tears are a complex mixture of fatty oils, water, mucus, and more than 1500 different proteins that keep the surface of the eye smooth and protected from the environment, irritants, and infectious pathogens. Tears include three layers:
- an outer, oily lipid layer, produced by the Meibomian glands, keeps tears from evaporating too quickly and helps tears remain on the eye;
- a middle aqueous layer, including lacrimal fluid produced by the main and accessory lacrimal glands, provides a watery physiologic saline to moisten and nourish the conjunctiva and cornea;
- an inner mucin layer, produced by goblet cells, binds water from the aqueous layer to ensure that the eye remains wet.

Artificial tear substitutes in the form of eye drops, eye gel, or eye spray are offered for treating dry eye. Artificial tear substitutes, however, might contain only some components of natural tears, and as such, might not provide a sustainable treatment for dry eye.

### SUMMARY

According to the present disclosure, eye drops for treating dry eye include serum extracted from whole blood. Advantageously, serum-based eye drops are biomechanically and biochemically similar to lacrimal fluid in the aqueous layer of tears. In contrast to artificial tears in current use, serum-based eye drops provide a more sustainable treatment for dry eye.

In some cases, serum eye drops may be produced from autologous whole blood, *i.e.,* whole blood obtained from the patient to be treated. In other cases, serum eye drops may be produced from allogenic whole blood, *i.e.,* whole blood obtained from a donor who is not the patient to be treated. The use of allogenic whole blood allows a patient who cannot provide his or her own whole blood to be treated with serum eye drops. A supply of eye drops for treating dry eye can be more conveniently produced and delivered to patients without requiring the patients to provide their own whole blood.

According to aspects of the present disclosure, an example method includes collecting whole blood from one or more donors (*e.g.,* allogenic whole blood); separating the whole blood to obtain a serum; applying radiation to the serum for viral inactivation; and adding one or more additives to the serum to produce eye drops.

The example method may further include selecting the whole blood from the one or more donors according to blood type.

In the example method, separating the whole blood serum may include coagulating the whole blood and centrifuging the whole blood.

The example method may further include pooling the serum from a plurality of donors to produce a desired volume of eye drops.

In the example method, the radiation may be corpuscular radiation and/or electromagnetic radiation.

The example method may further include testing the whole blood for particular viruses.

The example method may further include applying additional radiation to the serum and/or the eye drops to reduce a level of active biological contaminants or pathogens.

In the example method, separating the whole blood to obtain the serum may result in separation of cellular components from the serum.

In the example method, the one or more additives may supplement active components in the serum according to particular concentrations.

In the example method, the one or more additives may include one or more stabilizers that allow the eye drops to be stably stored at particular temperatures.

In the example method, the one or more additives may include one or more pharmaceuticals (*e.g*., cyclosporine).

In the example method, the one or more additives (*e.g*., saline and/or hyaluronic acid) may determine a viscosity of the eye drops.

The example method may further include storing the eye drops in packaging (*e.g.,* single dose containers). In this case, the example method may further include applying additional radiation to the stored eye drops for viral inactivation.

The example method may further include combining the eye drops with a medium that dissolves in an eye over time and releases the eye drops in a controlled and time-dependent manner.

One or more steps of the method may employ low temperature to slow degradation of the whole blood, serum, and/or eye drops.

### BRIEF DESCRIPTION OF THE DRAWINGS

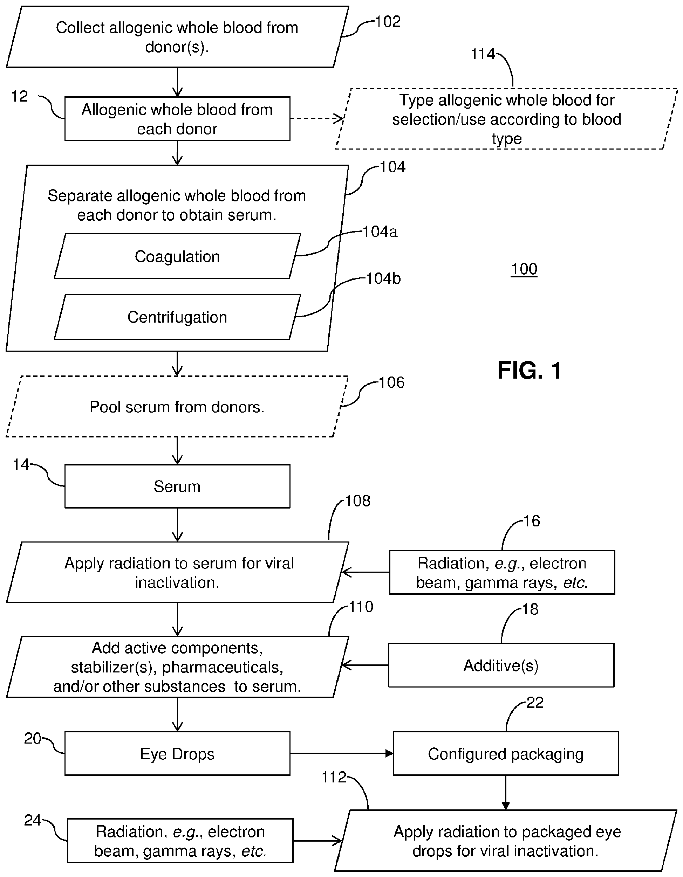

FIG. 1 illustrates an example process for producing eye drops from allogenic whole blood for treatment of dry eye, according to aspects of the present disclosure.

While the present disclosure is susceptible to various modifications and alternative forms, a specific embodiment thereof has been shown by way of example in the drawings and will herein be described in detail. It should be understood, however, that it is not intended to limit the present disclosure to the particular forms disclosed, but on the contrary, the intention is to cover all modifications, equivalents, and alternatives falling within the spirit of the present disclosure.

### DESCRIPTION

According to the present disclosure, eye drops for treating dry eye include serum extracted from whole blood. Advantageously, serum-based eye drops are biomechanically and biochemically similar to lacrimal fluid in the aqueous layer of tears. In contrast to artificial tears in current use, serum-based eye drops provide a more sustainable treatment for dry eye.

In some cases, serum eye drops may be produced from autologous whole blood, *i.e.,* whole blood obtained from the patient to be treated. In other cases, serum eye drops may be produced from allogenic whole blood, *i.e.,* whole blood obtained from a donor who is not the patient to be treated. The use of allogenic whole blood allows a patient who cannot provide his or her own whole blood to be treated with serum eye drops. A supply of eye drops for treating dry eye can be more conveniently produced and delivered to patients without requiring the patients to provide their own whole blood.

FIG. 1 illustrates an example process 100 for producing eye drops 20 from allogenic whole blood 12 for treatment of dry eye. In act 102, the allogenic whole blood 12 is collected from one or more donors who are not the patient to be treated. The allogenic whole blood 12 may be typed as A/B/AB/O +/- and selected in act 114 so that the eye drops 20 can be matched for use by patients according to blood type. In act 104, the allogenic whole blood 12 from each donor is separated to obtain serum 14. The allogenic whole blood 12 from each donor may be separated, for instance, by a process including coagulation in act 104a and centrifugation in act 104b.

To produce a desired supply of eye drops 20, allogenic whole blood 12 may be collected from a plurality of donors and the serum from each donor may be pooled in act 106 to provide the serum 14. Such pooling can provide a high volume of the eye drops 20 for a plurality of doses and/or distribution to a plurality of patients. For instance, a sufficient amount of serum 14 can be pooled from approximately sixteen donors, *i.e.,* the process 100 can produce a sufficient amount of serum 14 from sixteen donors on average. However, it is understood that any number of donors may be employed to produce the eye drops 20.

After the serum 14 has been separated from the rest of the allogenic whole blood 12 and, if necessary, pooled, radiation 16 is applied to the serum 14 in act 108 for viral inactivation. The radiation 16 provides a sufficient dose of energy to render viruses (*e.g*., enveloped/non-enveloped, DNA/RNA-based, *etc.*) in the serum 14 inactive. The radiation 16 may be corpuscular, *i.e.,* include beams of subatomic particles such as neutrons, electrons, and/or protons. Alternatively or additionally, the radiation 16 may be electromagnetic, *e.g*., include gamma rays. In some cases, the process 100 may include additional act(s) to test the allogenic whole blood 12 from each donor for certain viruses. If necessary, the allogenic whole blood 12 from a particular donor may be excluded or processed in other ways to ensure that such viruses are not present in the serum 14.

Radiation may also be applied to sterilize the serum 14, *i.e.,* reduce the level of active biological contaminants or pathogens. Although the allogenic whole blood 12 collected from the one or more donors may be initially sterile and the acts of the process 100 may be conducted to preserve this sterility, a sterilizing process with radiation may be additionally employed during the process 100. For instance, a sterilizing process may be applied before viral inactivation in act 108. This sterilizing process may employ corpuscular radiation (*e.g*., beams of subatomic particles such as neutrons, electrons, and/or protons) or electromagnetic radiation (*e.g*., radio waves, visible/invisible light, infrared light, ultraviolet radiation, x-radiation, and/or gamma rays). The type and dose of radiation to achieve viral inactivation, however, may be different from sterilizing application of radiation. The sterilizing process may also employ sensitizers that enhance the sterilizing effect of the radiation on the biological contaminants or pathogens. Additionally or alternatively, sterilizing radiation may be applied at another point in the process, *e.g.,* to sterilize the eye drops 20.

Exposure to the radiation 16 does not unacceptably damage the serum 14. For instance, after act 104, cellular components are separated from the serum 14, so that only proteins remain in the serum 14 as active ingredients. Such proteins are not unacceptably denatured by electron beams, gamma rays, or other forms of the radiation 16. The application of the radiation 16 may also include the use of substances that can further minimize any damage to the serum 14.

Once viral inactivation has been achieved in act 108, one or more additives 18 may be added to the treated serum 14 in act 110. For instance, the serum 14 may include one or more active components that determine the efficacy of the eye drops 20. As such, certain active components in the serum 14 can be supplemented in act 110 to enhance the efficacy of the eye drops 20. Indeed, the eye drops 20 can be distributed as various standard products based on the concentrations of the different active components. Potency assays may be employed to identify how active components can be supplemented to enhance efficacy.

Stabilizers may also be added in act 110 to allow for storage of the eye drops 20 at particular temperatures. For instance, the stabilizer(s) can allow the eye drops 20 to be stored stably for extended periods at room temperature, *i.e.,* approximately 70°F. Alternatively or additionally, the stabilizer(s) can allow the eye drops 20 to be stored stably for extended periods at temperatures below room temperature but without requiring freezing or deep cooling of the eye drops 20.

The serum 14 may also be combined in act 110 with one or more pharmaceuticals, which for instance may further assist in the treatment of dry eye. For instance, the serum 14 may be combined with pharmaceuticals, such as cyclosporine. Furthermore, other substances, excipients, *etc.,* may be added to the serum 14 in act 110 to provide the eye drops 20 with particular characteristics, *e.g*., for storage, delivery, application, combination with other substances (*e.g.* pharmaceuticals), *etc.* For instance, the viscosity of the eye drops 20 can be adjusted by adding saline, hyaluronic acid, *etc.*

Once any additives 18 have been added to the serum 14 in act 110, the resulting product can be implemented as eye drops 20. The eye drops 20 are stored in packaging 22 that is specially configured for delivering and optionally applying the eye drops 20 to patients. The packaging 22, for instance, may be single use containers (single dose containers) that ensure aseptic use of the eye drops 20 as a biologic.

In some cases, the eye drops 20 may be applied in a treatment with various time release techniques. For instance, the eye drops 20 may be applied via a medium that dissolves in the eye over time and releases the eye drops in a controlled and time-dependent manner.

Although the radiation 16 is applied in act 108 to the serum as a batch for viral inactivation, FIG. 1 shows that additional radiation 24 (electron beams, gamma rays, *etc.*) can be additionally applied in act 112 to the packaged eye drops 20 for viral inactivation. The type and amount of the radiation 24 applied in act 112 may be the same as or different from the radiation 16 applied in act 108. In alternative embodiments, however, viral inactivation can be achieved by applying the radiation 16 to the batch only, *e.g.,* act 108. In other alternative embodiments, viral inactivation can be achieved by applying the radiation 16 to the packaged eye drops only, *e.g.,* act 112.

As described above, one or more active components may determine the efficacy of the eye drops 20. Some active components may be susceptible to temperature degradation. As such, aspects of the process 100 may be conducted in the lowest possible temperatures and/or may employ various low temperature techniques to slow such degradation and to increase shelf life.

Although FIG. 1 may illustrate a series of acts in a particular order, it is understood that certain acts may occur more than once and/or in different orders. For instance, act 110 may occur several times to add different substances separately to the serum 14. Additionally, although the example process 100 shown in FIG. 1 produces the eye drops 20 from the serum 14, it is understood that the serum 14 can be used in other forms of treatments, such as eye sprays or eye gels. It is also contemplated, however, that the eye drops 20 may be employed as a more general excipient for the delivery of other types of pharmaceutical(s) for treatments of other disorders of the eye.

In view of the foregoing, aspects of the present disclosure can provide one or more of the following features:
- a high volume of serum for treating dry eye and/or other disorder, where the serum is pooled from a plurality of donors and processed to render viruses inactive;
- a serum-based eye treatment with certain (*e.g*., supplemented) concentrations of active components for enhanced efficacy in treatments of dry eye and/or other disorder;
- a serum-based eye treatment that can be stored stably at approximately room temperature, or at lower temperatures without requiring freezing or deep cooling;
- a serum than can include combined with one or more pharmaceuticals for treating dry eye and/or other disorder; or
- a serum-based eye treatment that is matched to the patient according to blood type.

While the present disclosure has been described with reference to one or more particular embodiments, those skilled in the art will recognize that many changes may be made thereto without departing from the spirit and scope of the present disclosure. Each of these embodiments and obvious variations thereof is contemplated as falling within the spirit and scope of the present disclosure. It is also contemplated that additional embodiments according to aspects of the present disclosure may combine any number of features from any of the embodiments described herein.
The following clauses, describing aspects of the invention, are part of the description:
WHAT IS CLAIMED IS:
1. A method, comprising:
   collecting whole blood from one or more donors;
   separating the whole blood to obtain a serum;
   applying radiation to the serum for viral inactivation; and
   adding one or more additives to the serum to produce eye drops.
2. The method of clause 1, further comprising selecting the whole blood from the one or more donors according to blood type.
3. The method of any of the preceding clauses, wherein separating the whole blood serum includes coagulating the whole blood and centrifuging the whole blood.
4. The method of any of the preceding clauses, further comprising pooling the serum from a plurality of donors.
5. The method of any of the preceding clauses, wherein the radiation is corpuscular radiation and/or electromagnetic radiation.
6. The method of any of the preceding clauses, further comprising testing the whole blood for particular viruses.
7. The method of any of the preceding clauses, further comprising applying additional radiation to the serum and/or the eye drops to reduce a level of active biological contaminants or pathogens.
8. The method of any of the preceding clauses, wherein separating the whole blood to obtain the serum results in separation of cellular components from the serum.
9. The method of any of the preceding clauses, wherein the one or more additives supplement active components in the serum according to particular concentrations.
10. The method of any of the preceding clauses, wherein the one or more additives include one or more stabilizers that allow the eye drops to be stably stored at particular temperatures.
11. The method of any of the preceding clauses, wherein the one or more additives include one or more pharmaceuticals.
12. The method of clause 11, wherein the one or more additives include cyclosporine.
13. The method of any of the preceding clauses, wherein the one or more additives determine a viscosity of the eye drops.
14. The method of clause 13, wherein the one or more additives include saline and/or hyaluronic acid.
15. The method of any of the preceding clauses, further comprising storing the eye drops in packaging.
16. The method of clause 15, further comprising applying additional radiation to the stored eye drops for viral inactivation.
17. The method of clause 15, wherein the packaging includes single dose containers.
18. The method of any of the preceding clauses, further comprising combining the eye drops with a medium that dissolves in an eye over time and releases the eye drops in a controlled and time-dependent manner.
19. The method of any of the preceding clauses, wherein one or more steps of the method employ low temperature to slow degradation of the whole blood, serum, and/or eye drops.
20. Eye drops produced according to any of the preceding clauses.

## Claims

1. A method, comprising:
collecting whole blood from a plurality of donors;
separating the whole blood from each donor to obtain a serum from each donor;
pooling the serums from the plurality of donors to obtain a pooled serum after pooling the serum, applying radiation to the pooled serum for viral inactivation; and
after applying radiation to the pooled serum, adding one or more additives to the pooled serum to supplement active components and produce eye drops having particular concentrations of active components of the serum.

2. The method of claim 1, further comprising selecting the whole blood from the one or more donors according to blood type.

3. The method of any of the preceding claims, wherein separating the whole blood serum includes coagulating the whole blood and centrifuging the whole blood.

4. The method of any of the preceding claims, wherein the radiation is corpuscular radiation and/or electromagnetic radiation.

5. The method of any of the preceding claims, further comprising testing the whole blood for particular viruses.

6. The method of any of the preceding claims, further comprising applying additional radiation to the serum and/or the eye drops to reduce a level of active biological contaminants or pathogens.

7. The method of any of the preceding claims, wherein separating the whole blood to obtain the serum results in separation of cellular components from the serum.

8. The method of any of the preceding claims, wherein the one or more additives include one or more stabilizers that allow the eye drops to be stably stored at particular temperatures.

9. The method of any of the preceding claims, wherein the one or more additives include one or more pharmaceuticals and wherein the one or more additives include cyclosporine.

10. The method of any of the preceding claims, wherein the one or more additives determine a viscosity of the eye drops and wherein the one or more additives include saline and/or hyaluronic acid.

11. The method of any of the preceding claims, further comprising storing the eye drops in packaging and further comprising applying additional radiation to the stored eye drops for viral inactivation and wherein the packaging includes single dose containers.

12. The method of any of the preceding claims, further comprising combining the eye drops with a medium that dissolves in an eye over time and releases the eye drops in a controlled and time-dependent manner.

13. The method of any of the preceding claims, wherein one or more steps of the method employ low temperature to slow degradation of the whole blood, serum, and/or eye drops.
